# EUROPEAN PATENT SPECIFICATION

(11) **EP 2 248 507 B1**
(45) Date of publication and mention of the grant of the patent: **09.01.2013**
(21) Application number: 10003125.1
(22) Date of filing: 24.03.2010
(51) Int. Cl.: A61J 15/00

(54) **Medical fluid container**
Behälter für medizinische Flüssigkeiten
Conteneur de fluide médical

(30) Priority: 07.05.2009 US 436871
(43) Date of publication of application: 10.11.2010
(73) Proprietor: Covidien LP, Mansfield, MA 02048 (US)
(72) Inventor: Fitzgerald, Alan, Edgeworthstown Co. Longford (IE)
(74) Representative: Tomlinson, Edward James

(56) References cited:
- EP-A1- 1 795 169
- WO-A1-95/24177
- US-A- 4 713 064
- US-A- 4 795 429
- US-A- 4 856 995
- US-A- 5 916 201
- US-A1- 2007 287 966

## Description

### FIELD OF THE INVENTION

The present invention generally relates to medical fluid containers and more specifically to a medical fluid container with more than one compartment.

### BACKGROUND OF THE INVENTION

In many medical situations, one or more medical fluids are administered to a patient. Administering fluids containing medicine or nutrients to a patient is well known in the art. For example, patients that are unable to consume enough food or fluids by mouth to meet their nutritional needs may need to receive nourishment by an enteral feeding system. Although the medical fluid container of the present invention may be used in various medical situations, the container will be discussed herein primarily in the context of enteral feeding.

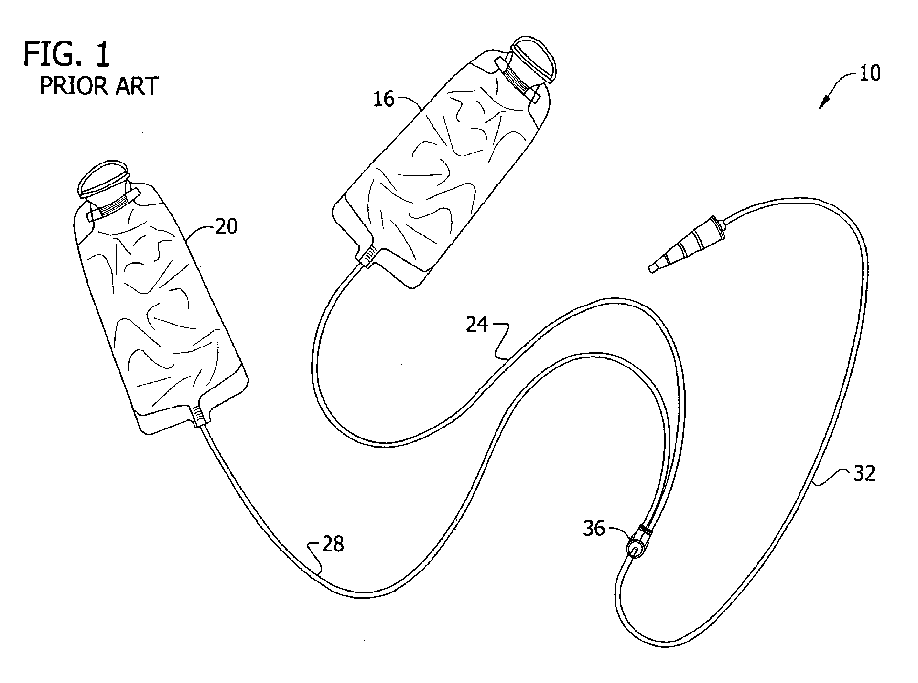

U.S. Patent No. 7,462,170, which is assigned to Covidien AG and herein incorporated by reference in its entirety, discloses an enteral or administration feeding set, which is illustrated in Fig. 1 and generally designated 10. The administration feeding set 10 comprises two separate fluid sources 16, 20, which are generally fluid-filled bags (also designated 16, 20). The first bag 16 contains liquid food or nutrients, and the second bag 20 contains flushing fluid (e.g., water) for rinsing the feeding set or for hydrating the patient. The bags 16, 20 are made of flexible, non-rigid material (e.g., relatively thin plastic film) that is not self-supporting.

Fluid from the bags 16, 20 is delivered to a patient by respective source tubes 24, 28 and a delivery tube 32. The source tubes 24, 28 are joined in fluid communication with the delivery tube 32 by a valve mechanism 36 or by a simple Y-connection (not shown).

Before the beginning of a feeding session, the liquid food bag 16 and the rinsing fluid bag 20 are connected to respective source tubes 24, 28. As necessary, liquid food is delivered to the patient through the first source tube 24 and the delivery tube 32, and rinsing fluid is delivered to the patient for hydration through the second source tube 28 and the delivery tube. When the feeding session is complete, the second source tube 28 and the delivery tube 32 may be cleaned by flushing the tubes with rinsing fluid from the rinsing fluid bag 20.

The administration feeding set 10 has certain drawbacks. For example, the first source tube 24 cannot be flushed with fluid from the rinsing fluid bag 20. If the first source tube 24 is not otherwise cleaned, liquid food may remain in the first source tube for an extended period of time, leading to blockage and contamination of the tube. In addition, the tubing 24, 28, 32 may become tangled or kinked and result in ineffective administration of medical fluids. Further, the several pieces of tubing 24, 28, 32 and the valve mechanism 36 require assembly and add to the cost of manufacture. For at least these reasons, there is a need for a simplified and improved medical fluid container for use with such enteral feeding systems and for other medical fluid applications.

EP 1 795 169 A1 describes an enteral feeding container having two compartments separated by a dividing wall. A rotatable valve is used to control which compartment is connected to an outlet.

### SUMMARY OF THE INVENTION

One aspect of the present invention as claimed is directed to a medical fluid container comprising a single reservoir of fluid impermeable material, the reservoir having a fluid
impermeable barrier dividing the reservoir into at least first and second separate fluid-holding compartments;
a first fluid flow path from the first compartment of the reservoir to said fluid delivery tube;
a second fluid flow path from the second compartment of the reservoir to the fluid delivery tube; and
a valve selectively movable from a first position allowing flow of a fluid along said first flow path from the first compartment but blocking flow of fluid from the second compartment along said second flow path, to a second position allowing flow of a fluid along said second flow path from the second compartment but blocking flow of fluid from the first compartment along said first flow path,
where the valve comprises a valve housing and a valve member
movable in the valve housing, said first flow path comprising a first flow passage in the valve member, and said second flow path comprising a second flow passage in the valve member separate from the first flow passage, said valve housing further comprising a connector for connecting said fluid delivery line to the valve housing, said connector having a releasable connection with the valve housing and wherein said valve member is a spool mounted for sliding movement in a cavity in the valve housing, said spool having a first end portion, a second end portion , and an intermediate portion between the end portions, and wherein said first flow passage is located between the first end portion and the intermediate portion of the spool, and the second flow passage is located between the second end portion and the intermediate portion of the spool.

The invention is also directed to a method of dispensing medical fluids from a medical fluid container. The container comprises a single reservoir of fluid impermeable material. The reservoir has a fluid impermeable barrier dividing the reservoir into at least first and second compartments holding first and second medical fluids, respectively. The method comprises moving a valve to a first position in which the valve permits flow of the first medical fluid from the first compartment, and in which the valve blocks flow of the second medical fluid from the second compartment.

Other objects and features will be in part apparent and in part pointed out hereinafter.

### BRIEF DESCRIPTION OF THE DRAWINGS

Fig. 1 is a perspective of a prior art medical fluid delivery set;

Fig. 2 is a front elevation of a medical fluid container of the present invention;

Fig. 3 is a right side elevation of the medical fluid container of Fig. 2;

Fig. 4 is a left elevation of the medical fluid container of Fig. 2;

Fig. 5 is a front perspective of the medical fluid container;

Fig. 6 is an exploded front perspective of the medical fluid container;

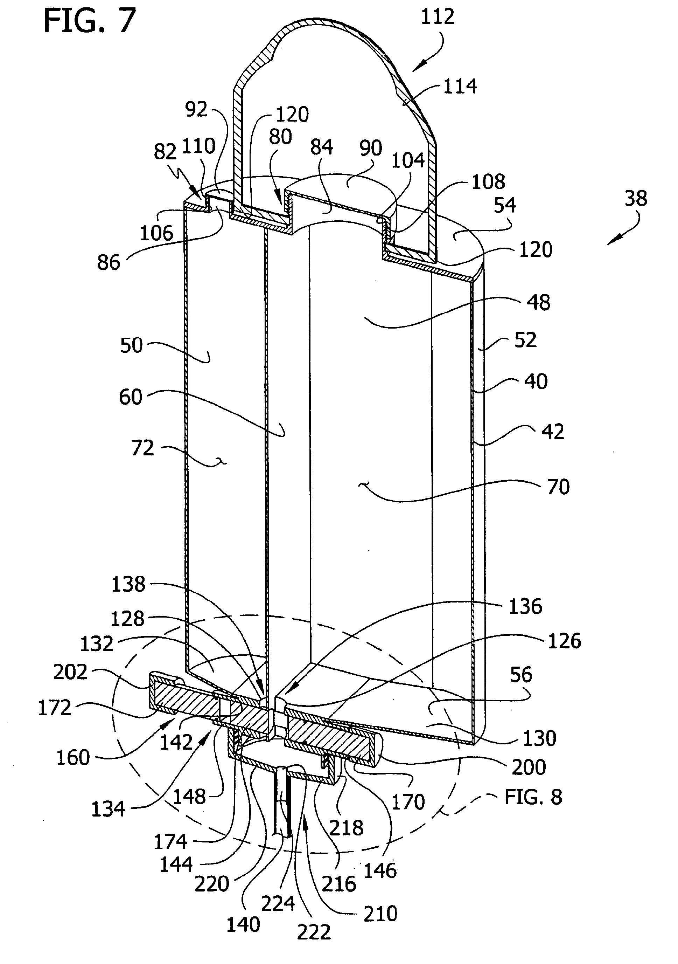

Fig. 7 is a vertical section of the medical fluid container in the plane of line 7--7 of Fig. 5;

Fig. 8 is an enlarged vertical section of a lower portion of the medical fluid container as indicated in Fig. 7;

Fig. 9 is horizontal section of the medical fluid container in the plane of line 9-9 of Fig. 2;

Fig. 10 is an enlarged perspective of a valve spool of a valve of the medical fluid container;

Fig. 11 is a plan view of the valve spool;

Fig. 12 is an enlarged vertical section of the valve spool in the plane of line 12-12 of Fig. 11;

Fig. 13 is an enlarged vertical section of the lower portion of the medical fluid container in the plane of line 13--13 of Fig. 2;

Fig. 14A is a vertical section of the lower portion of the medical fluid container, similar to the view of Fig. 7, showing the valve in a first position;

Fig. 14B is a view similar to Fig. 14A showing the valve in a second position;

Fig. 14C is a view similar to Fig. 14A and 14BB showing the valve in a third position;

Fig. 15 is a front perspective of another embodiment of the medical fluid container.

Corresponding reference characters indicate corresponding parts throughout the drawings.

### DETAILED DESCRIPTION

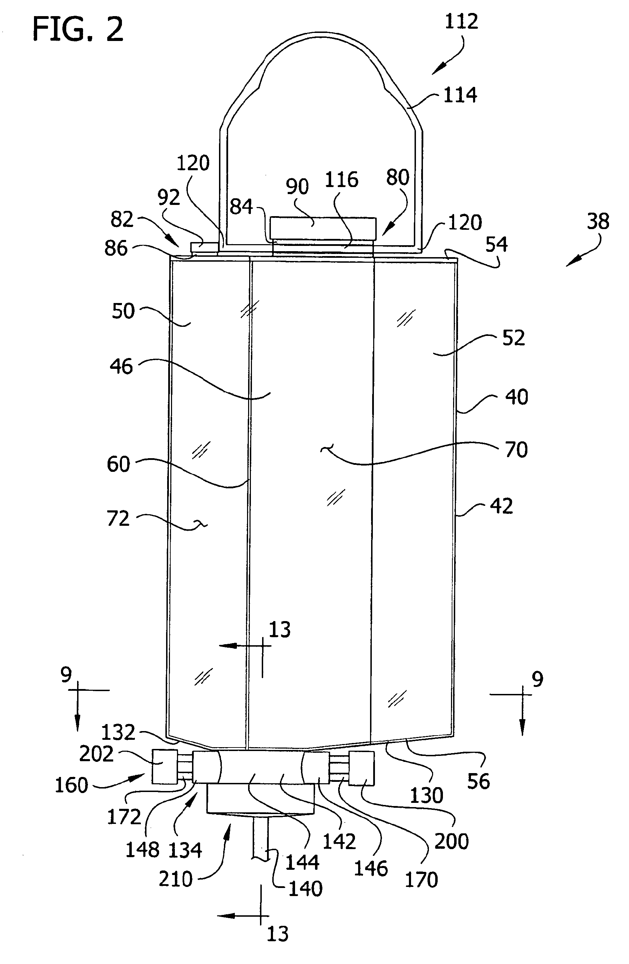

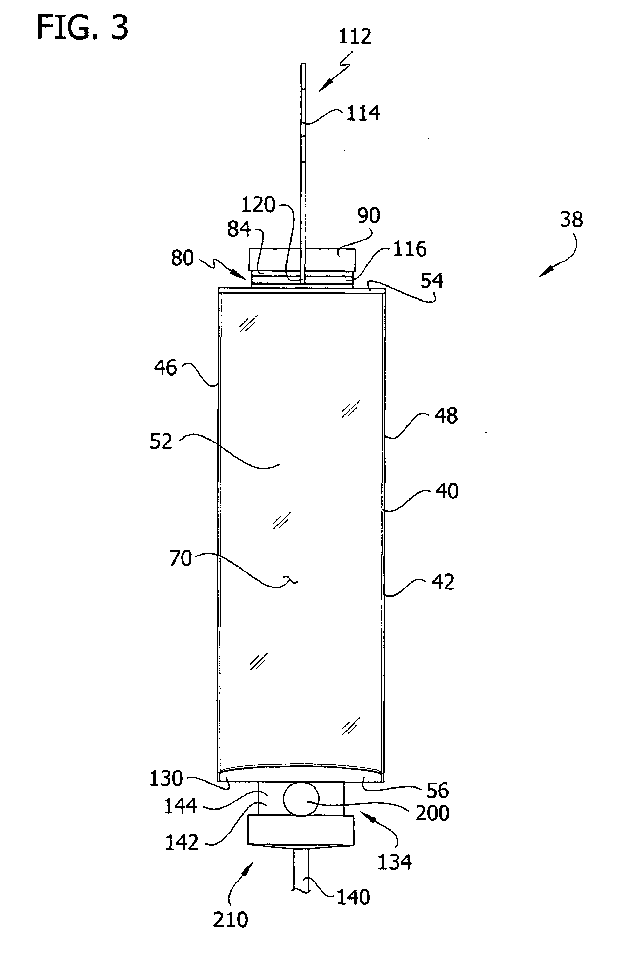

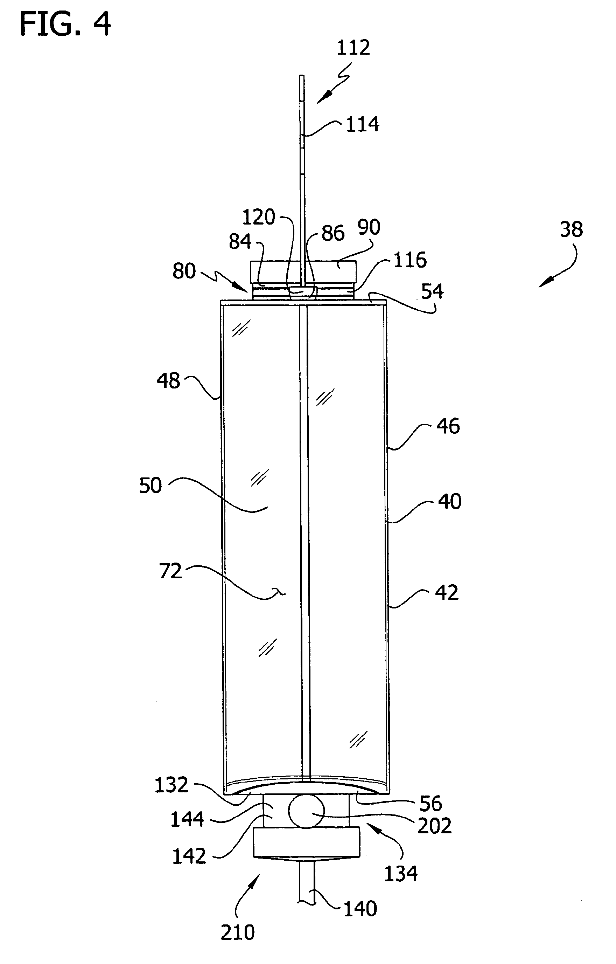

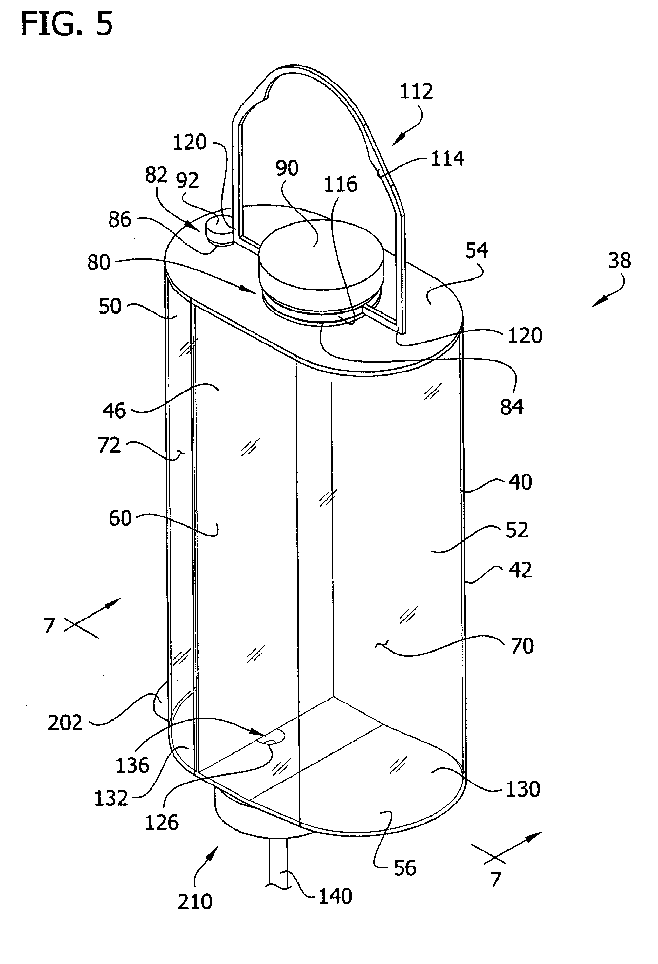

Referring to the drawings, Figs. 2-5 show a first embodiment of a medical fluid container 38 of this invention that may be used to administer one or more medical fluids to a patient. Such medical fluids may contain medicine or nutrients. For example, the container 38 may be used to administer medical fluid to a patient intravenously. Alternatively, the container 38 may be used to deliver nourishment (e.g., liquid food and water) to a patient by enteral feeding. The medical fluid container 38 may be used in a gravity feed only system or in a system having a pump (e.g., a peristaltic pump) to deliver medical fluids from the container to a patient. The container 38 is configured to hold medical fluids separately within the container to prevent mixing of the fluids. Other uses for the medical fluid container 38 are within the scope of the present invention.

The medical fluid container 38 comprises a single reservoir of fluid impermeable material, generally designated 40. Desirably, the reservoir 40 is made of a substantially rigid and self-supporting material, such as a rigid plastic, and is formed by a molding operation. The rigid plastic is desirably translucent, semi-transparent or transparent to enable viewing of medical fluid within the container 38. A main body 42 of the illustrated reservoir 40 has a generally oval or racetrack shape and comprises substantially planar front 46 and back 48 walls and curved substantially semi-cylindrical left 50 and right 52 side walls. The reservoir further comprises a top wall 54 and a bottom wall 56, which are described in more detail below. Reservoirs of different shapes are within the scope of the present invention.

A fluid impermeable barrier 60 divides the reservoir into first 70 and second 72 separate fluid-holding compartments and prevents medical fluid held within the compartments from mixing. The fluid impermeable barrier 60 of the illustrated embodiment comprises an interior wall or partition (also designated 60). Desirably, the partition 60 is also formed of a substantially rigid and self-supporting material, such as rigid plastic, and may be formed in the same molding operation as the main body 42 of the reservoir 40. Alternatively, the partition 60 may be formed separately and later inserted into the main body 42. In either case, the peripheral edges of the partition 60 desirably have fluid tight seals with respective inside surfaces of the reservoir 40 to define the first compartment 70 on one side of the partition and the second compartment 72 on the opposite side of the partition. One or more fluid impermeable barriers 60 may be used to divide the reservoir 40 into two or more (e.g., three, four, or five) fluid-holding compartments. The one or more barriers 60 prevent fluid in one compartment 70, 72 from mixing with fluid in another compartment.

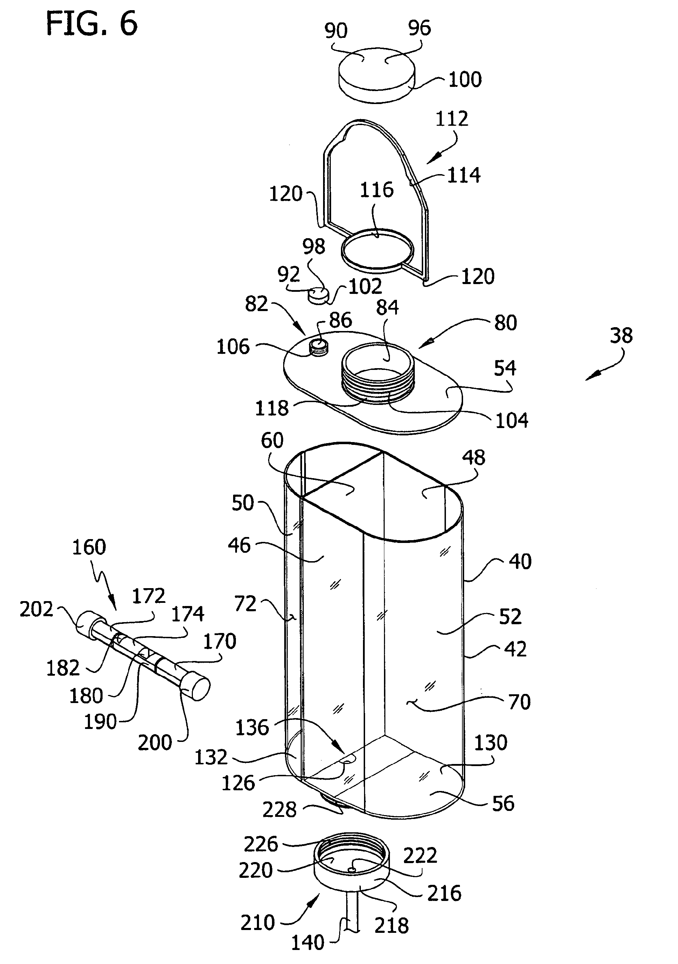

As shown in Fig. 6, the fluid impermeable barrier 60 is configured and positioned within the reservoir 40 to define the desired volume of each fluid-holding compartment 70, 72. In different applications for which the medical fluid container 38 may be used, the container may desirably need to be capable of holding certain ratios of two or more fluids. For example, the container 38 may need to hold more of a first fluid than a second fluid. In the illustrated embodiment, the barrier 60 divides the reservoir 40 in a 10:1 1 ratio such that the volume of the first compartment 70 is approximately ten times the volume of the second compartment 72. Other ratios may be used as desired, including 1:1, 2:1, 3:1, 4:1 and so forth.

The fluid impermeable barrier 60 extends vertically from the top to the bottom of the reservoir 40 in a plane generally perpendicular to the front 46 and back 48 walls of the container 38. However, the barrier 60 may have other configurations. By way of example, the barrier 60 may be oriented in a non-vertical or slanted plane and may be non-perpendicular to the front 46 and back 48 walls. Furthermore, the barrier 60 may be configured such that different portions of the barrier lie in different planes (e.g., vertical, horizontal, or slanted) within the reservoir 40. The barrier 60 may also have a non-planar (e.g., curved) shape. However, the barrier 60 is desirably configured such that fluid held within the two compartments 70, 72 readily moves by gravity from the upper end to the lower end of the container 38 and does not "pool" against surfaces of the barrier or the reservoir 40.

The top wall 54 of the reservoir defines first and second inlets 80, 82 through which the two fluid-holding compartments 70, 72 of the container 38 may be filled. The inlets 80, 82 are positioned on the top wall 54 and overlie portions of the reservoir 40 on opposite sides of the partition 60 such that the first inlet 80 provides access to the first compartment 70 but not the second compartment 72, and the second inlet 82 provides access to the second compartment but not the first compartment. Thus, fluid may be introduced into the first and second compartments 70, 72 independently. In the illustrated embodiment, the inlets 80, 82 comprise annular necks 84, 86, respectively, formed as one piece with and extending up from the upper surface of the top wall 54. The first inlet 80 is shown having a larger diameter than the second inlet 82, but other relative sizes may be used.

First and second caps 90, 92 are configured for releasable connection to the first and second inlets 80, 82, respectively. The caps 90, 92 are removable to enable introduction of medical fluid into the compartments 70, 72 as necessary. The caps 90, 92 each comprise a top wall 96, 98 and an annular side wall or skirt 100, 102 (Fig. 6). Threads 104, 106 are formed on an exterior surface of the necks 84, 86 for threaded connection with mating threads 108, 110 on an inside surface of the annular side walls 100, 102 of the caps 90, 92. The caps 90, 92 desirably form a fluid-tight seal over the inlets 80, 82 to prevent fluid held within the compartments from escaping through the inlets when the caps are threaded on the inlets. The caps 90, 92 also shield the inlets 80, 82 to prevent undesirable contaminants from entering the compartments 70, 72 through the inlets. The caps 90, 92 may be color coded such that the color of the cap associated with one of the compartments 70, 72 corresponds to the color assigned to the medical fluid designated to be held within that compartment. Inlets of different shapes and sizes, as well as different types of caps (e.g., snap-fit), are within the scope of the present invention.

A support 112 connected to the container 38 is adapted for supporting the container in a generally vertical orientation. The support 112 may be rigidly fixed to the container 38 or be pivotally connected to the container such that the container may swivel about the connection to maintain a vertical orientation. In the illustrated embodiment, the support 112 comprises a handle or hanger (also designated 112) of rigid plastic. The hanger 112 comprises a loop 114 and a ring 116. Two ends of the loop 114 are connected to the ring 116 at diametrically opposite sides of the ring. The ring 116 is fitted over the neck 84 of the first inlet 80 and positioned in an annular recess or groove 118 (Fig. 6) to restrain the ring from vertical movement on the neck. The ring 116 is rotatable about the inlet neck 84 such that the hanger 112 may be rotated 360 degrees about a vertical axis of the inlet 80. Because the first inlet 80 is generally centered over the upper end of the container 38, the hanger 112 supports the container in a generally vertical orientation when used to hang the container or when held by hand as a handle. Clearance is desirably provided between the support 112 and the inlets 80, 82 such that the support does not interfere with filling of the container 38. For example, right angles 120 are formed near the ends of the loop 114 so that the ends of the loop extend outward in radial directions from the ring 116 to provide clearance for an operator to engage the side wall 100 of the first cap 90 and rotate it by hand. Supports of different configurations are within the scope of the present invention. For example, the support 112 may be connected to the top wall 54 in a different manner or at a different location on the container 38, and the support may comprise a hook instead of a loop 114.

The container 38 has first and second outlets 126, 128 in fluid communication with the first and second fluid-holding compartments 70, 72. The outlets 126, 128 are located at the lower end of the container 38 on opposite sides of the partition 60 such that fluid held within the container may move by gravity to the outlets. Portions 130, 132 of the bottom wall 56 of the reservoir 40 are canted or sloped toward the outlets 126, 128 to promote gravity flow of fluids held within the container 38 to respective outlets. Desirably, the flow area of the first outlet 126 is greater than the flow area of the second outlet 128. The larger first outlet 126 allows more fluid flow from the first compartment 70 than the smaller outlet 128 allows from the second compartment 72. Other relative sizes and shapes of the outlets 126, 128 are within the scope of the present invention. In the illustrated embodiment, the outlets 126, 128 are formed by a single opening that is divided by the partition 60 to define two outlets. Alternatively, the outlets 126, 128 can be formed by two separate openings on opposite sides of the partition.

A valve, generally designated 134, is mounted below the reservoir for controlling flow of fluid from the fluid-holding compartments 70, 72 along first and second separate flow paths 136, 138 to a fluid delivery tube 140. As described in more detail below, the valve 134 is selectively movable to first, second and third positions to allow flow from either of the two compartments 70, 72 or to block flow from the compartments. Moreover, the valve 134 is adapted for repeated selected movement to the first, second and third positions.

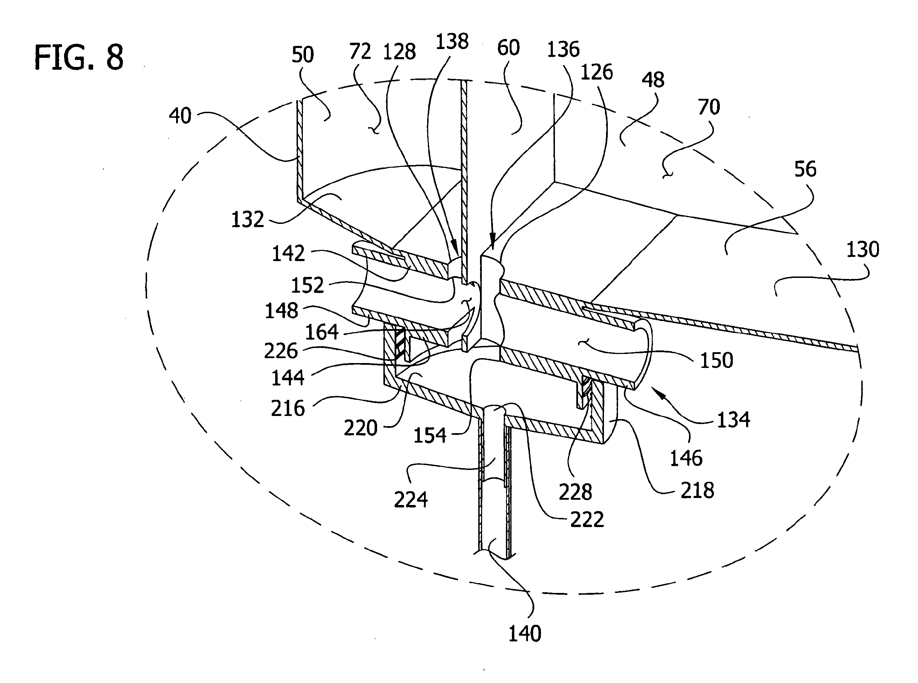

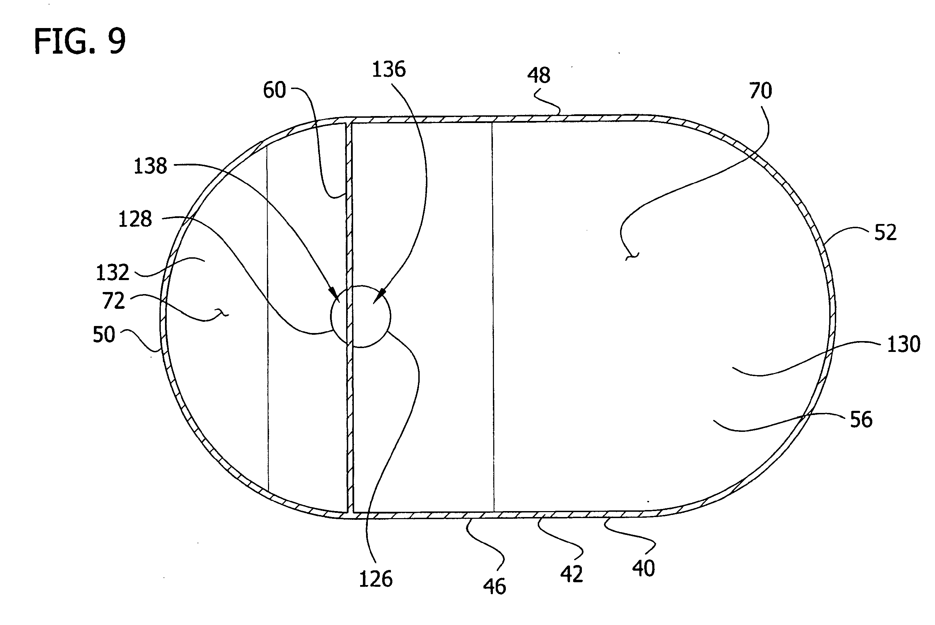

In the illustrated embodiment, the valve 134 comprises a housing 142 integrally formed with the bottom wall 56 of the reservoir 40. The housing 142 includes a cylindrical central portion 144 and aligned sleeve portions 146, 148 extending laterally from opposite sides of the central portion. The central portion 144 and sleeves 146, 148 combine to define a cylindrical cavity 150 (Fig. 8) that is open at both ends. As shown in Figs. 8 and 9, the valve housing 142 has an inlet 152 in fluid communication with the outlets 126, 128 in the reservoir 40 and an outlet 154 for exit of fluid from the housing. The partition 60 extends down through the valve housing 142 from the inlet 152 to the outlet 154 and divides the valve housing inlet and outlet into sections corresponding to respective flow paths 136, 138. Desirably, the reservoir outlets 126, 128, housing inlet 152 and housing outlet 154 are in substantially vertical alignment for efficient fluid flow.

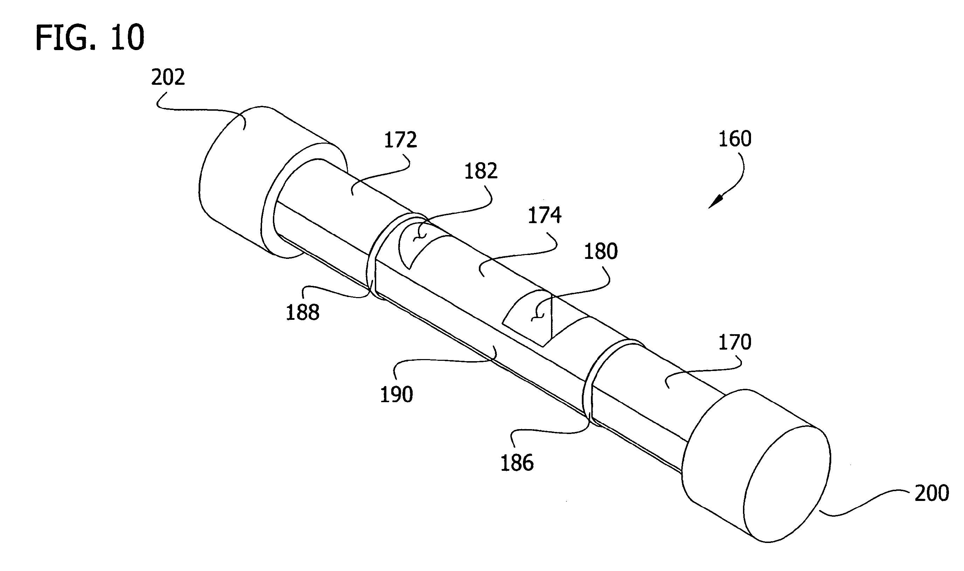

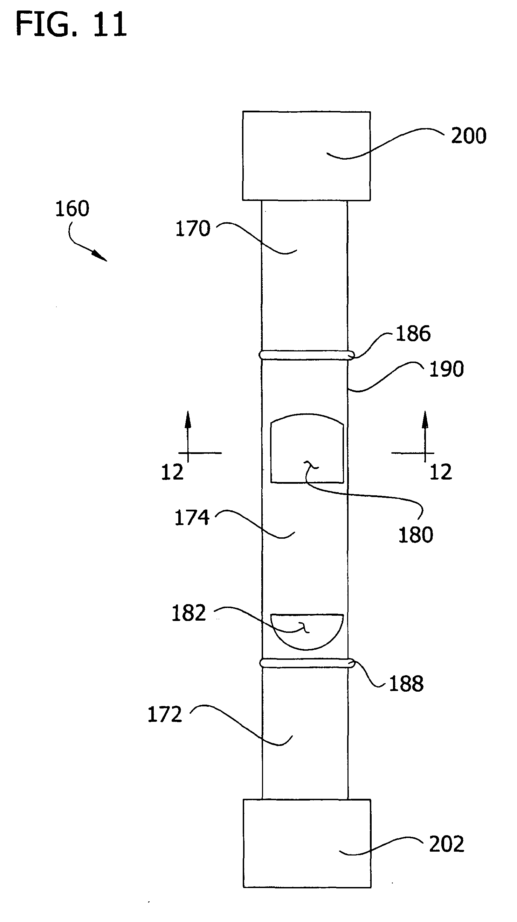

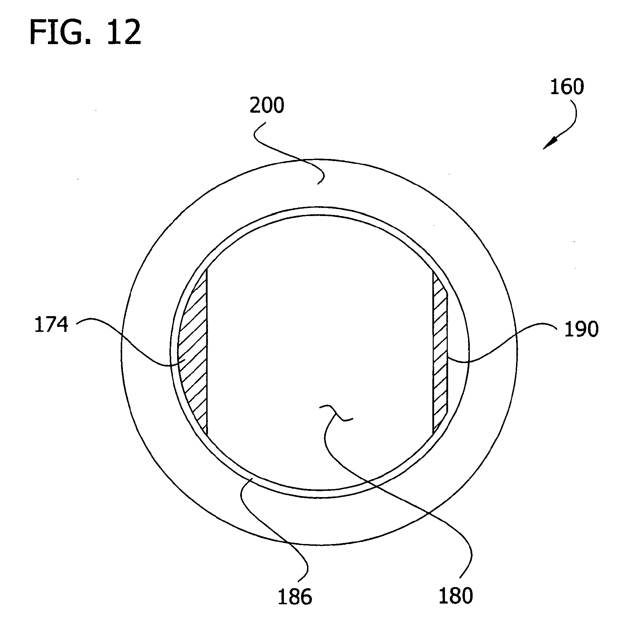

The valve 134 also includes an elongate valve member comprising a spool 160 mounted for sliding movement in the cavity 150 through an aligned opening 164 (Fig. 8) in the partition 60 along an axis generally at right angles to the central axis of the outlet opening 154. As shown in Figs. 10-12, the valve spool 160 comprises a first end portion 170, a second end portion 172, and an intermediate portion 174 between the end portions. The spool 160 also has two flow passages 180, 182 formed by through-holes extending generally at right angles to the longitudinal axis of the spool at locations spaced axially of the spool. The first flow passage 180 is located between the first end portion 170 and the intermediate portion 174 of the spool 160, and the second flow passage 182 is located between the second end portion 172 and the intermediate portion of the spool. O-ring seals 186, 188 are provided around the end portions 170, 172 of the spool 160 outboard of respective flow passages 180, 182 to seal against the wall of the cavity 150 in the housing 142 to prevent leakage of fluid from the ends of the cavity.

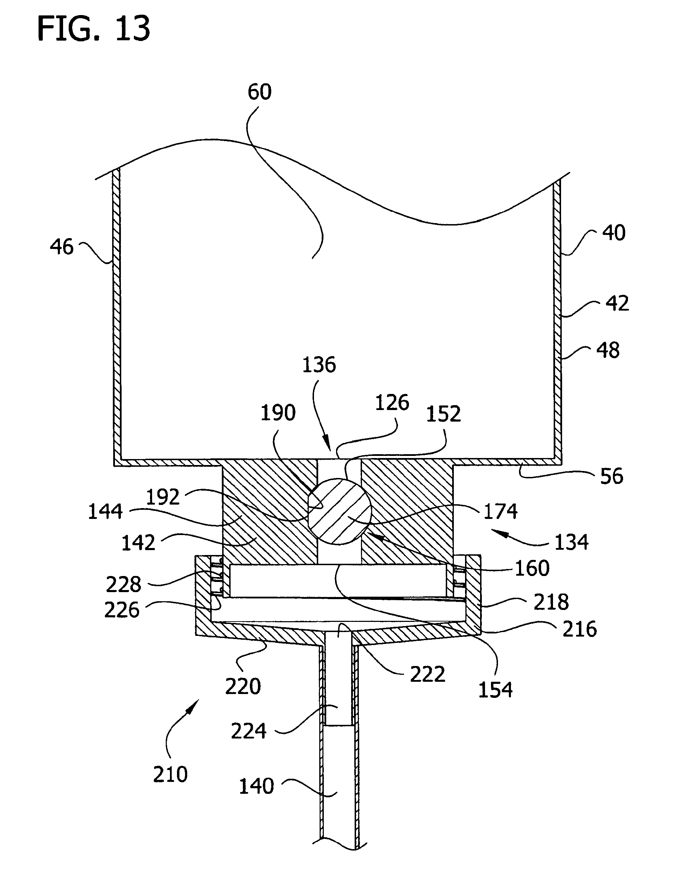

The valve spool 160 is configured to slide in the housing 142 along the longitudinal axis of the cavity 150. However, the spool 160 is held against rotation in the housing 142 so that the flow passages 180, 182 remain in a fixed angular orientation as the spool slides back and forth. The spool 160 is held against such rotation by one or more flats on the spool in engagement with one or more corresponding flats on the cavity wall. In the illustrated embodiment, the spool 160 has a flat 190 extending substantially the entire length of the spool for contacting a flat 192 on the wall of the cavity 150, as shown in Fig. 13. Other mechanisms may be used to prevent rotation of the spool 160 in the cavity 150. Alternatively, the flow passages 180, 182 in the spool valve 160 can be formed by circumferential grooves (not shown) instead of through-holes, in which case there may be no need to hold the spool valve against rotation.

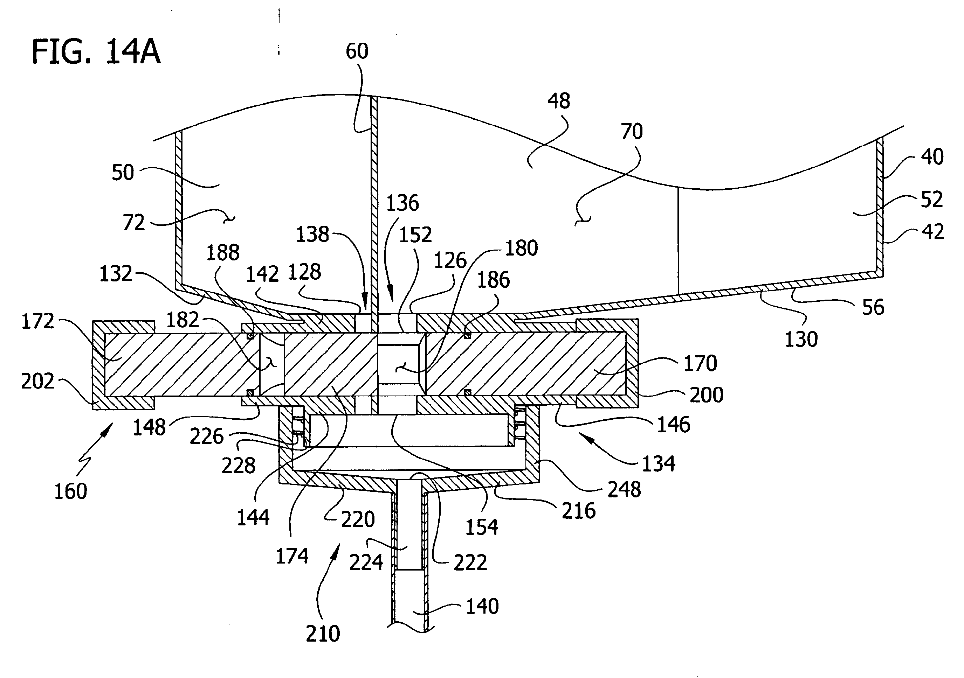

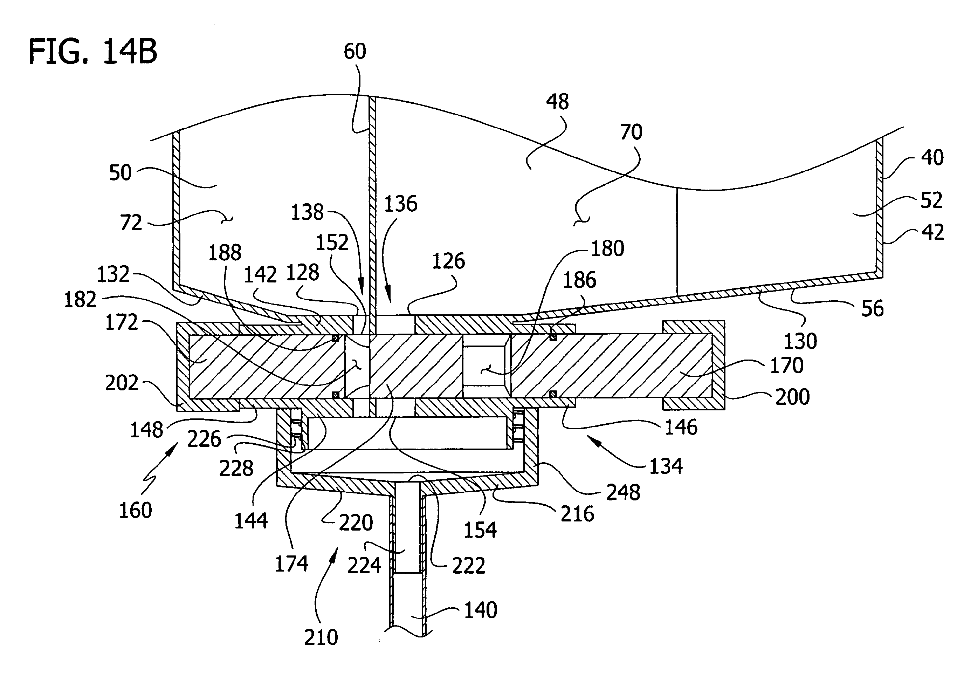

First and second stops 200, 202 are provided on the first and second end portions 170, 172 of the spool 160 to limit the axial sliding movement of the spool valve to positions allowing fluid flow from one or the other compartment 70, 72 of the reservoir 40. In particular, and as best illustrated in Fig. 14A, the first stop 200 limits axial sliding movement of the spool 160 in the housing 142 in one direction (to the left) to a position in which the first flow passage 180 in the spool communicates with the first outlet 126 of the reservoir but not with the second outlet 128, and the second flow passage 182 of the spool is out of communication with both outlets 126, 128. As a result, fluid is able to flow from the first compartment 70 but not the second compartment 72. As best illustrated in Fig. 14B, the second stop 202 limits axial sliding movement of the spool 160 in the housing 142 in the opposite direction (to the right) to a position in which the second flow passage 182 of the spool 160 communicates with the second outlet 128 but not the first outlet 126, and the first flow passage 180 of the spool is out of communication with both outlets 126, 128. As a result, fluid is able to flow from the second compartment 72 but not from the first compartment 70. The flow area of the first flow passage 180 in the spool 160 is greater than the flow area of the second flow passage 182 in the spool to accommodate more flow through the first outlet 126 of the reservoir 40 than the second outlet 128 of the reservoir, as noted previously. This relative sizing of the flow passages 180, 182 in the spool 160 can be varied depending on the desired flow rates from respective compartments 70,72.

In the illustrated embodiment, each stop 200, 202 is in the shape of a cap (also designated 200, 202) which fits on a respective end 170, 172 of the spool 160. However, other stop devices can be used to limit the movement of the spool 160 between the desired first and second positions.

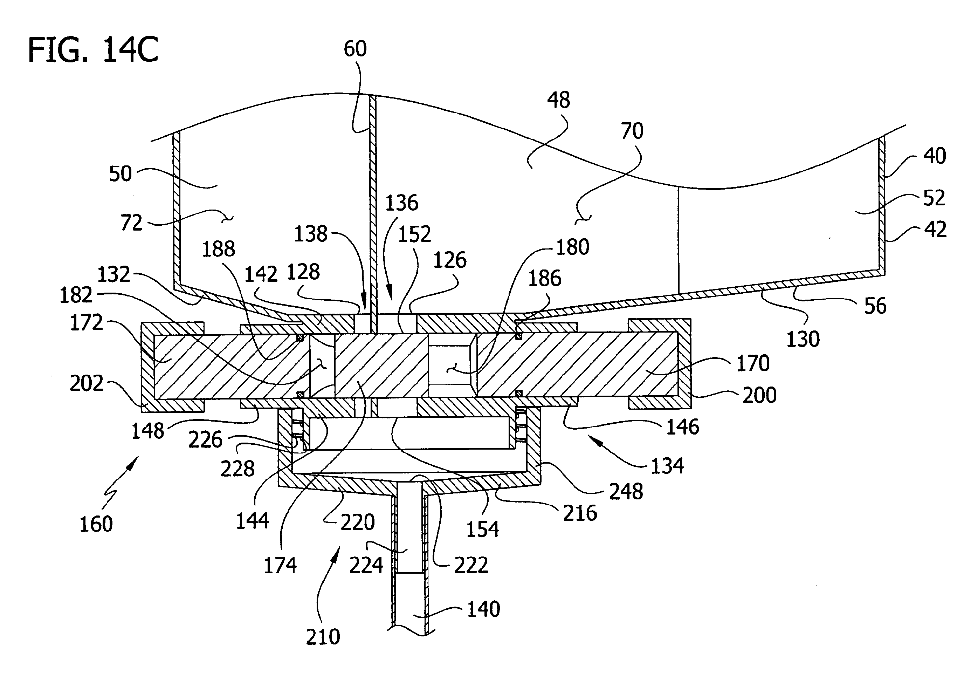

The valve spool 160 can also be selectively moved to a third position blocking flow from either compartment 70, 72, as shown in Fig. 14C. In this position, the spool 160 is moved to a position in which the passages 180, 182 are not in communication with either outlet 126, 128 and the intermediate portion 174 of the spool 160 closes the inlet 152 and outlet 154 of the valve housing 142. The fit between the intermediate spool portion 174 and partition 60 is desirably a sealing fit or near-sealing fit to minimize fluid cross-over between the flow paths 136, 138 on opposite sides of the partition.

The container 38 also includes a tube connector, generally designated 210, for connecting the fluid delivery tube 140 to the valve housing 142 for flow of fluid from the outlet 154 of the valve housing to the tube 140. The tube connector 210 comprises a funnel-shaped member 216 having an annular side wall 218, a bottom wall 220 with an opening 222, and a connecting tube 224 extending down from the opening for connection to the delivery tube 140. The connection may be a sealing press fit, for example, in which the connecting tube 224 is received in an open end of the delivery tube 140. The tube connector 210 has a releasable connection with the valve housing 142. In the illustrated embodiment, the connection is a threaded connection between threads 226 on the annular wall 218 of the funnel-shaped member 216 and threads 228 on the central portion 144 of the valve housing 142. The arrangement is such that when the valve 134 is in its first position (Fig. 14A), fluid is able to flow along the first flow path 136 comprising the first reservoir outlet 126, valve housing inlet 152, spool valve flow passage 180, valve housing outlet 154, and connecting tube 224 into the delivery tube 140. Similarly, when the valve 134 is in its second position (Fig. 14B), fluid is able to flow along the second flow path 138 comprising the second reservoir outlet 128, valve housing inlet 152, spool valve flow passage 182, valve housing outlet 154, and connecting tube 224 into the delivery tube 140. The bottom wall 220 of the funnel-shaped member 216 is sloped to drain all fluid into the connecting tube 224.

Other valves used to control flow through the outlet from the fluid-holding compartments are within the scope of the present invention. For example, a rotary valve may be selectively movable by rotation of a rotor to allow flow of a fluid from either of the two fluid-holding compartments 70, 72 or to block flow from the compartments.

The first and second flow paths 136, 138 can have configurations different from the configurations shown in the above embodiment. For example, as noted previously, the flow passages 180, 182 through the valve member 160 (e.g., spool 160) can be defined by through-holes of different sizes and shapes, and also by grooves. Alternatively, the flow paths 136, 138 could be defined by changing the position of a single flow passage in the valve member 160, as in a rotary valve arrangement. Further, the valve housing 142 and/or fluid delivery tube connector 210 can have other shapes and configurations which may affect the configuration of the flow paths 136, 138. Also, additional structure could be added between the reservoir outlets 126, 128 and the valve housing 142 and/or between the valve housing and the fluid delivery tube connector 210.

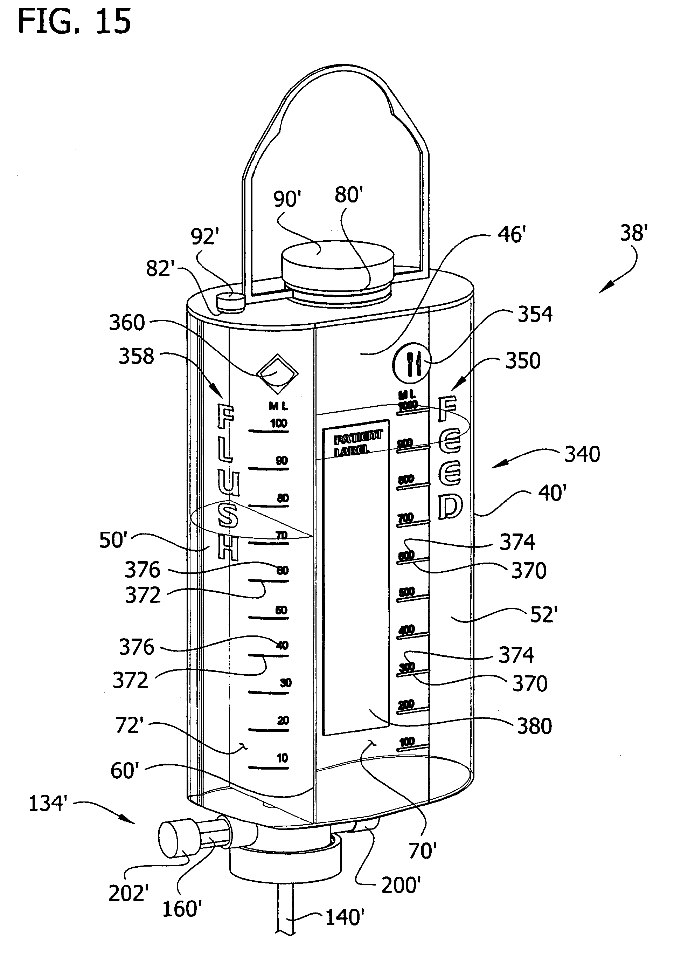

Fig. 15 illustrates a second embodiment of a medical fluid container, generally designated 38'. The container 38' is similar in most respects to the container 38 described above, and corresponding parts are designated by the corresponding reference numbers, plus a prime designator ('). In this embodiment, the medical fluid container 38' has indicators or markings, generally designated 340, for indicating information regarding use of the container. For example, the container 38' has indicators (e.g., text or symbols) 340 for designating that the compartments are for holding a particular type of medical fluid. The indicators 340 are desirably displayed (e.g., printed) on the container 38' at locations adjacent or overlying the compartments 70', 72' in which related medical fluids are designated to be held.

In the embodiment shown in Fig. 15, the medical fluid container 38' is for enteral feeding. The first compartment 70' is adapted for holding liquid food, and the second compartment 72' is adapted for holding rinse fluid. The word "FEED" 350 is printed on the side wall 52' of the container 38' overlying the first compartment 70' to indicate that the compartment is designated for holding liquid food. A circular symbol 354 with a knife and fork is also printed overlying the first compartment 70' for indicating that the compartment is designated for holding liquid food. Similarly, the word "FLUSH" 358 is printed on the side wall 50' of the container 38' overlying the second compartment 72' for indicating that the compartment is designated for holding flush or rinse fluid. A diamond-shaped symbol 360 having an internal shape resembling a droplet of fluid (e.g., water) is also printed overlying the second compartment 72' for indicating that the compartment is designated for holding flush or rinse fluid. Other such text, symbols, or other indicators 340 used for signifying liquid food, rinse fluid, or any other medical fluid are within the scope of the present invention.

The medical fluid container 38' also has indicators or markings 340 for assisting in measurement of the volume of fluid held in the compartments 70', 72'. The container 38' is marked at sections overlying each of the fluid-holding compartments 70', 72' by lines 370, 372 and associated numbers 374, 376 to indicate the milliliters (ML) of fluid held within each of the compartments. Because of the substantially rigid and self-supporting nature of the container 38', the measurement indicators 370-376 provide a relatively accurate means for measuring the volume of fluid within the compartments 70', 72', as compared to the relatively poor accuracy of measurement indicators on non-rigid or flexible bags. In the illustrated embodiment, the lines 370 for the first or liquid food compartment 70' are spaced at increments of 100 ML and indicate volumes ranging from 100 to 1000 ML. The lines 372 for the second or rinsing fluid compartment 72' are spaced at increments of 10 ML and indicate volumes ranging from 10 to 100 ML. The first compartment 70' is shown holding approximately 900 ML of liquid food, and the second compartment 72' is shown holding approximately 70 ML of water. The illustrated container 38' is configured to hold more liquid food than rinsing fluid because more liquid food is generally necessary for an enteral feeding session. Other units of measurement and/or measurement increments may be used. Additionally, the total volume of each compartment 70', 72' may be varied as desired according to the size of the reservoir 40' and the configuration and orientation of the partition 60' dividing the reservoir into separate compartments.

The informational indicators or markings 340 may be color coded to correspond to the type of medical fluid designated to be held within respective compartments 70', 72'. The caps 90', 92' over the inlets 80', 82' and the stops 200', 202' on the valve spool 160' may be color coded. Similarly, the measurement indicators 370-376, the text 350, 358, and/or the symbols 354, 360, may be color coded. Desirably, all colored components that relate to a particular medical fluid are color coded according to a color assigned to that medical fluid. For example, in the embodiment shown in Fig. 15, the color purple is used to designate liquid food or feed, and the color green is used to designate rinsing or flush fluid. Thus, the cap 90', stop 200', measurement indicators 370, 374, and other informational indicators 350, 354 associated with the first compartment 70' are color coded purple. The cap 92', stop 202', measurement indicators 372, 376, and other informational indicators 358, 360 associated with the second compartment 72' are color coded green. More or fewer components may be color coded, and other colors may be used.

An indicator 380 may be secured to or printed on the container 38' to provide information relating to the patient for whom the medical fluid container is designated. For example, the name of a patient or other information relating to the patient may be printed or written directly onto the container (e.g., on the front wall 46') or on a label (also designated 380) secured to the container 38'.

The medical fluid container 38 may be different than described above and still be within the scope of the present invention. For example, the container 38 may have an entirely different shape. The outlet 126 and the inlets 180 may be on surfaces other than the top 54 and bottom 56 walls, respectively, of the container 38. The container 38 may have more than one impermeable barrier or partition 60 that divides the reservoir 40 into two or more compartments 70, 72. In such a configuration, the outlet 126 would be suitably configured to allow fluid flow through the outlet from the compartments.

As compared to medical fluid delivery sets such as the set 10 shown in Fig. 1 and described above in the Background, the medical fluid container 38 presents a simplified and improved solution. For example, the medical fluid container 38, having a single outlet 126 in fluid communication with separate compartments 70, 72 of the container, eliminates the need for two source tubes 24, 28 extending from separate fluid bags 16, 20 and a valve 36 or other connection between the two source tubes and the delivery tube 32. Further, the medical fluid container 38 allows for a simplified cleaning process. The rinsing fluid is held upstream of and may thus be flushed through all of the tubing 340 used to deliver a fluid to a patient.

In use, the container 38 is prepared for a medical fluid delivery session by filling the container with one or more desired medical fluids. Before filling, the valve spool 160 is slid to the third position, as shown in Fig. 14C, such that the intermediate portion 174 of the valve spool 160 is vertically aligned with both channels 156, 158 in fluid communication with the compartments 70, 72. Thus, flow of fluid from either of the compartments 70, 72 through the outlet 126 is blocked in preparation for filling the compartments. The hanger or other support 112 may be used to maintain the container 38 in a generally vertical orientation while filling the compartments 70, 72. The caps 90, 92 are removed from respective inlets 80, 82 to allow introduction of medical fluids into the compartments 70, 72. One or both of the caps 90, 92 may be removed from the inlets 80, 82 to fill one or both of the compartments 70, 72, as necessary.

If particular medical fluids are designated for one or both of the compartments 70, 72, care should be taken to fill the compartments with the proper medical fluid. If a medical fluid container 38' as shown in Fig. 15 is used, this may be done by reference to, for example, color coding of components (e.g., caps 90', 92' and stops 200', 202') associated with the compartments 70', 72' or text 350, 358, symbols 354, 360 or other indicators 340. The compartments 70', 72' may be filled partially or completely with a desired volume of medical fluid by reference to respective measurement indicators 370-376 on the container 38'. The caps 90, 92 are threaded back onto the inlets 80, 82 once the compartments 70, 72 have been filled with medical fluid as desired. The delivery tube 140 may be connected to the container 38 before or after filling the compartments 70, 72 with medical fluid by threading the delivery tube connector 210 onto the central portion 144 of the valve housing 142.

A medical fluid delivery session may then be started. The delivery tube 140 is first administered to the patient. The medical fluid may be administered to the patient by gravity only flow, in which case the hanger or other support 112 may be used to support the container 38 above the point of delivery to the patient. Alternatively, a pump may be used. For example, the delivery tube 140 may be loaded onto a peristaltic pump (not shown) to move fluid from the container 38 to the patient. In either case, the container 38 is desirably maintained in a generally vertical orientation by use of the support 112 or otherwise such that medical fluid within the compartments 70, 72 may move by gravity to the outlet 126.

Delivery of a medical fluid is initiated by selectively moving the valve 134 to either the first position or the second position, as shown in Figs. 14A and 14B, respectively. One of the stops 200, 202 may be pushed to slide the valve spool 160. If medical fluid held within the first compartment 70 is to be delivered, the spool 160 is moved to the first position (Fig. 14A) such that the first passage 180 is in communication with the first reservoir outlet 126 to allow fluid flow from the first compartment. In this valve position, the second reservoir outlet 128 is out of communication with both valve flow passages 180, 182 thus blocking fluid flow from the second compartment 72. The stop 200 abuts against the sleeve portion 146 of the valve housing 142 when the first passage 180 communicates with the first reservoir outlet 126.

If medical fluid held within the second compartment 72 is to be delivered, the valve spool 160 is moved to the second position (Fig. 14B) such that the second passage 182 is in communication with the second reservoir outlet 128 to allow fluid flow from the second compartment. In this valve position, the first reservoir outlet 126 is out of communication with both valve flow passages 180, 182 thus blocking fluid flow from the first compartment 70. The stop 202 abuts against the sleeve portion 148 of the valve housing 142 when the second passage 182 communicates with the second reservoir outlet 128.

The valve 134 may be repeatedly selectively moved to the first, second and third positions to permit and block fluid flow from the compartments 70, 72 according to the desired sequence and volume of medical fluid to be delivered to the patient. Reference may be made to the measurement indicators 370-376 to deliver the desired volume of each medical fluid. If either of the compartments 70, 72 needs to be refilled during the medical fluid delivery session, it may be filled by following the above-described steps of sliding the valve shaft 146 to the third ("no-flow") position, removing the respective cap 90, 92, and filling the compartment 70, 72. A specific desired amount may be filled by reference to the measurement indicators 370-376. Alternatively, the compartments 70, 72 may be refilled when the valve 134 is in the first or second positions. However, if it is desired to fill a compartment 70, 72 with a specified volume of fluid, the compartment should desirably be filled when the valve 134 is blocking fluid flow from that compartment. For example, the first compartment 70 and not the second compartment 72 should be filled when the valve 134 is in the second position (Fig. 14B). The compartments 70, 72 may be filled without removing the delivery tube 140 from the container 38 or removing the delivery tube from the patient.

Upon completion of the medical fluid delivery session, the valve spool 160 is slid to the third ("no-flow") position (Fig. 14C), and the delivery tube 140 is removed from the patient. If a rinsing fluid is held within one of the compartments 70, 72, the delivery tube 140 may be cleaned by flushing the rinsing fluid through the tube. The delivery tube 140 is desirably positioned over a drain, and the valve spool 160 is slid to a position (e.g., the second position of Fig. 14B) in which the rinsing fluid may flow through the delivery tube. Rinsing fluid may be flushed through the delivery tube 140 by gravity only or by use of a pump. Once the delivery tube 140 is sufficiently clean, the valve spool 160 is moved back to the third position (Fig. 14C) to block flow from either of the compartments 70, 72 through the valve 134. Alternatively, the delivery tube 140 may be removed from the container 38 for cleaning by unthreading the delivery tube connector 210 from the central portion 144 of the valve housing 142.

If the medical fluid container 38' is for enteral feeding, as shown in the embodiment of Fig. 15, the first compartment 70' desirably contains liquid food, and the second compartment 72' desirably contains rinsing fluid. The valve 134' is moved to the first position (Fig. 14A) in which the liquid food is permitted to flow from the first compartment 70' to a patient, and in which flow of the rinsing fluid is blocked. The valve 134' may then be moved to the second position (Fig. 14B) to permit flow of rinsing fluid from the second compartment 72'. In this valve position, flow of the liquid food through the outlet 126' is blocked. The valve 134' may be moved to the second position to hydrate the patient with rinsing fluid (e.g., water) and/or to flush the delivery tube 140' connected to the outlet 126'.

Having described the invention in detail, it will be apparent that modifications and variations are possible without departing from the scope of the invention defined in the appended claims.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above constructions and methods without departing from the scope of the invention, it is intended that all matter contained in the above description and shown in the accompanying drawings shall be interpreted as illustrative and not in a limiting sense.

## Claims

1. A medical fluid container (38) adapted for connection to a fluid delivery tube, said container comprising:
a single reservoir (40) of fluid impermeable material, the reservoir having a fluid impermeable barrier (60) dividing the reservoir into at least first and second separate fluid-holding compartments (70, 72);
a first fluid flow path from the first compartment of the reservoir to said fluid delivery tube;
a second fluid flow path from the second compartment of the reservoir to the fluid delivery tube; and
a valve (1 34) selectively movable from a first position allowing flow of a fluid along said first flow path from the first compartment but blocking flow of fluid from the second compartment along said second flow path, to a second position allowing flow of a fluid along said second flow path from the second compartment but blocking flow of fluid from the first compartment along said first flow path,
**characterized in that** the valve comprises a valve housing (142) and a valve member (160) movable in the valve housing, said first flow path comprising a first flow passage in the valve member, and said second flow path comprising a second flow passage in the valve member separate from the first flow passage, said valve housing further comprising a connector (210) for connecting said fluid delivery line to the valve housing, said connector having a releasable connection with the valve housing and wherein said valve member is a spool mounted for sliding movement in a cavity in the valve housing, said spool having a first end portion (170), a second end portion (172), and an intermediate portion (174) between the end portions, and wherein said first flow passage is located between the first end portion and the intermediate portion of the spool, and the second flow passage is located between the second end portion and the intermediate portion of the spool.

2. A medical fluid container as recited in claim 1 wherein the valve is adapted for repeated selected movement between the first position, the second position, and a third position blocking flow of a fluid from the compartments.

3. A medical fluid container as recited in any of claims 1 or 2 wherein said first flow path comprises a first outlet in the reservoir on a first side of the barrier corresponding to the first compartment and the second flow path comprises a second outlet in the reservoir on a second side of the barrier corresponding to the second compartment, wherein the flow area of the first outlet is larger than the flow area of the second outlet.

4. A medical fluid container as recited in any of claims 1 to 3 wherein the reservoir and the fluid impermeable barrier are substantially rigid and self-supporting, wherein the fluid impermeable barrier comprises a partition having fluid tight seals with inside surfaces of the reservoir to define the first compartment on one side of the partition and the second compartment on an opposite side of the partition.

5. A medical fluid container as recited in any of claims 1 to 4 further comprising a first inlet (80) for the first compartment and a second inlet (82) for the second compartment such that fluid may be introduced into the first and second compartments independently.

6. A medical fluid container as recited in any of claims 1 to 5 wherein the container has at least one indicator (340) for designating that one of the compartments is for holding a particular type of medical fluid.

7. A medical fluid container as recited in any of claims 1 to 6 wherein the container is for enteral feeding, the first compartment being adapted for holding liquid food, and the second compartment being adapted for holding rinse fluid, and wherein a first indicator (350, 354) designates that the first compartment is for holding liquid food, and a second indicator (358, 360) designates that the second compartment is for holding rinse fluid.

8. A medical fluid container as recited in any of claims 1 to 7 further comprising measurement indicators (340) on at least one of the first and second compartments for indicating the volume of fluid in the compartment.

9. A medical fluid container as recited in any of claims 1 to 8 in combination with a delivery tube (140) and further comprising a support (112) connected to the container and adapted for supporting the container in a generally vertical orientation.

10. A medical fluid container as recited in any of claims 1 to 9 wherein components of the container associated with one of the first and second compartments are colour coded according to a colour assigned to a medical fluid designated to be held within respective compartments.

11. A method of dispensing medical fluids from a medical fluid container (38), said container comprising a single reservoir of fluid impermeable material (40), the reservoir having a fluid impermeable barrier (60) dividing the reservoir into at least first and second compartments (70, 72) holding first and second medical fluids, respectively, the method comprising:
pushing a stop (200, 202) to axially slide a spool (160) of a valve (134) to a first position in which the valve permits flow of said first medical fluid from the first compartment, and in which the valve blocks flow of said second medical fluid from the second compartment.

12. A method as recited in claim 11 further comprising pushing a stop (200, 202) to axially slide the spool (160) of the valve (134) to a second position in which the valve permits flow of said second medical fluid from the second compartment, and in which the valve blocks flow of said first medical fluid from the first compartment.

13. A method as recited in claim 12 wherein said first medical fluid is liquid food and said second medical fluid is rinse fluid, and wherein said method further comprises moving the valve to said first position to deliver liquid food to a patient and then later moving the valve to said second position to flush a delivery tube connected to said outlet.

14. A method as recited in claim 11 further comprising sliding the spool (160) of the valve (134) in a cavity (150) through an aligned opening (164) in the barrier (60) along an axis generally at a right angle to a central axis of an outlet (154) of the valve to said first position.

## Patentansprüche

1. Behälter (38) für medizinische Flüssigkeiten zur Verbindung mit einem Flüssigkeitsabgabeschlauch, wobei der Behälter Folgendes umfasst:
ein einzelnes Reservoir (40) aus flüssigkeitsundurchlässigem Material, wobei das Reservoir eine flüssigkeitsundurchlässige Barriere (60) aufweist, die das Reservoir in zumindest erste und zweite getrennte Flüssigkeit enthaltende Fächer (70, 72) trennt;
einen ersten Strömungsweg vom ersten Fach des Reservoirs zum Flüssigkeitsabgabeschlauch;
einen zweiten Strömungsweg vom zweiten Fach des Reservoirs zum Flüssigkeitsabgabeschlauch; und
ein Ventil (134), das selektiv von einer ersten Stellung, in der Flüssigkeit auf dem ersten Strömungsweg vom ersten Fach fließen kann, aber Flüssigkeitsströmung aus dem zweiten Fach auf dem zweiten Strömungsweg blockiert wird, in eine zweite Stellung, in der Flüssigkeit auf dem zweiten Strömungsweg aus dem zweiten Fach fließen kann, aber Flüssigkeitsströmung aus dem ersten Fach auf dem ersten Strömungsweg blockiert wird, bewegt werden kann
**dadurch gekennzeichnet, dass** das Ventil ein Ventilgehäuse (142) und ein im Ventilgehäuse bewegliches Ventilglied (160) umfasst, wobei der erste Strömungsweg einen ersten Strömungsdurchgang im Ventilglied umfasst und der zweite Strömungsweg einen vom ersten Strömungsweg getrennten zweiten Strömungsdurchgang im Ventilglied umfasst, wobei das Ventilgehäuse ferner ein Verbindungsglied (210) zur Verbindung der Flüssigkeitsabgabeleitung mit dem Ventilgehäuse umfasst, wobei das Verbindungsglied eine lösbare Verbindung mit dem Ventilgehäuse aufweist und worin das Ventilglied eine zur gleitenden Bewegung in einem Hohlraum im Ventilgehäuse angeordnete Spule ist, wobei die Spule einen ersten Endabschnitt (170), einen zweiten Endabschnitt (172) und einen Zwischenabschnitt (174) zwischen den Endabschnitten aufweist, und worin der erste Strömungsdurchgang zwischen dem ersten Endabschnitt und dem Zwischenabschnitt der Spule angeordnet ist und der zweite Strömungsdurchgang zwischen dem zweiten Endabschnitt und dem Zwischenabschnitt der Spule angeordnet ist.

2. Behälter für medizinische Flüssigkeit nach Anspruch 1, worin das Ventil für eine wiederholte ausgewählte Bewegung zwischen der ersten Stellung, der zweiten Stellung und einer dritten Stellung, in der Flüssigkeitsströmung aus den Fächern blockiert wird, ausgelegt ist.

3. Behälter für medizinische Flüssigkeit nach einem der Ansprüche 1 oder 2, worin der erste Strömungsweg einen ersten Auslass im Reservoir auf einer ersten Seite der Barriere entsprechend dem ersten Fach umfasst und der zweite Strömungsweg einen zweiten Auslass im Reservoir auf einer zweiten Seite der Barriere entsprechend dem zweiten Fach umfasst, worin der Strömungsbereich des ersten Auslasses größer als der Strömungsbereich des zweiten Auslasses ist.

4. Behälter für medizinische Flüssigkeit nach einem der Ansprüche 1 bis 3, worin das Reservoir und die flüssigkeitsundurchlässige Barriere im Wesentlichen starr und selbsttragend sind, worin die flüssigkeitsundurchlässige Barriere eine Trennwand mit flüssigkeitsdichten Abdichtungen mit Innenseiten des Reservoirs zur Definition des ersten Fachs auf einer Seite der Trennwand und des zweiten Fachs auf der gegenüberliegenden Seite der Trennwand umfasst.

5. Behälter für medizinische Flüssigkeit nach einem der Ansprüche 1 bis 4, der ferner einen ersten Einlass (80) für das erste Fach und einen zweiten Einlass (82) für das zweite Fach umfasst, so dass Flüssigkeit unabhängig in das erste und das zweite Fach eingeführt werden kann.

6. Behälter für medizinische Flüssigkeit nach einem der Ansprüche 1 bis 5, worin der Behälter zumindest einen Indikator (340) aufweist, der darauf hinweist, dass eines der Fächer zur Aufnahme einer bestimmten Art von medizinischer Flüssigkeit bestimmt ist.

7. Behälter für medizinische Flüssigkeit nach einem der Ansprüche 1 bis 6, worin der Behälter zur enteralen Ernährung bestimmt ist, wobei das erste Fach flüssige Nahrung aufnehmen kann und das zweite Fach eine Spülflüssigkeit aufnimmt und worin ein erster Indikator (350, 354) darauf hinweist, dass das erste Fach zur Aufnahme von flüssiger Nahrung bestimmt ist und ein zweiter Indikator (358, 360) darauf hinweist, dass das zweite Fach zur Aufnahme von Spülflüssigkeit bestimmt ist.

8. Behälter für medizinische Flüssigkeit nach einem der Ansprüche 1 bis 7, der ferner Messindikatoren (340) auf zumindest einem der ersten und zweiten Fächer zum Hinweis auf das Flüssigkeitsvolumen im Fach umfasst.

9. Behälter für medizinische Flüssigkeit nach einem der Ansprüche 1 bis 8 in Kombination mit einem Abgabeschlauch (140) und ferner einen mit dem Behälter verbundenen Träger (112) zur Unterstützung des Behälters in einer allgemein vertikalen Orientierung umfassend.

10. Behälter für medizinische Flüssigkeit nach einem der Ansprüche 1 bis 9, worin mit einem der ersten und zweiten Fächer in Zusammenhang stehende Bauteile des Behälters farbig kodiert sind je nach einer Farbe, die einer medizinischen Flüssigkeit zugewiesen wird, die in den jeweiligen Fächern aufgenommen werden soll.

11. Verfahren zur Abgabe von medizinischen Flüssigkeiten aus einem Behälter (38) für medizinische Flüssigkeiten, wobei der Behälter ein einzelnes Reservoir (40) aus flüssigkeitsundurchlässigem Material umfasst, wobei das Reservoir eine flüssigkeitsundurchlässige Barriere (60) aufweist, die das Reservoir in zumindest erste und zweite erste bzw. zweite medizinische Flüssigkeiten enthaltende Fächer (70, 72) trennt, wobei das Verfahren Folgendes umfasst:
Drücken eines Anschlags (200, 202) zum axialen Verschieben einer Spule (160) eines Ventils (134) in eine erste Stellung, in der das Ventil Strömung der ersten medizinischen Flüssigkeit aus dem ersten Fach gestattet und in der das Ventil Strömung der zweiten medizinischen Flüssigkeit aus dem zweiten Fach blockiert.

12. Verfahren nach Anspruch 11, das ferner das Drücken eines Anschlags (200, 202) zum axialen Verschieben der Spule (160) des Ventils (134) in eine zweite Stellung, in der das Ventil Strömung der zweiten medizinischen Flüssigkeit aus dem zweiten Fach gestattet und in der das Ventil Strömung der ersten medizinischen Flüssigkeit aus dem ersten Fach blockiert.

13. Verfahren nach Anspruch 12, worin die erste medizinische Flüssigkeit flüssige Nahrung ist und die zweite medizinische Flüssigkeit eine Spülflüssigkeit ist und worin das Verfahren ferner die Bewegung des Ventils in die erste Stellung zur Abgabe von flüssiger Nahrung an einen Patienten und später die Bewegung des Ventils in die zweite Stellung zum Durchspülen eines mit dem Auslass verbundenen Abgabeschlauchs umfasst.

14. Verfahren nach Anspruch 11, das ferner das Verschieben der Spule (160) des Ventils (134) in einen Hohlraum (150) durch eine fluchtende Öffnung (164) in der Barriere (60) entlang einer zur einer Mittelachse eines Auslasses (154) des Ventils allgemein rechtwinkligen Achse in die erste Stellung umfasst.

## Revendications

1. Conteneur (38) de fluide médical conçu pour être relié à un tube de distribution de fluide, ledit conteneur comportant :
un seul réservoir (40) en un matériau imperméable aux fluides, le réservoir étant pourvu d'une barrière (60) imperméable aux fluides qui divise le réservoir en au moins un premier et un deuxième compartiments (70, 72) séparés contenant un fluide ;
une première voie d'écoulement d'un fluide du premier compartiment du réservoir audit tube de distribution de fluide ;
une deuxième voie d'écoulement d'un fluide du deuxième compartiment du réservoir au tube de distribution de fluide ; et
une soupape (134) sélectivement mobile entre une première position permettant l'écoulement d'un fluide dans ladite première voie d'écoulement depuis le premier compartiment mais qui bloque l'écoulement d'un fluide depuis le deuxième compartiment dans ladite deuxième voie d'écoulement, et une deuxième position permettant l'écoulement d'un fluide dans ladite deuxième voie d'écoulement depuis le deuxième compartiment mais qui bloque l'écoulement d'un fluide depuis le premier compartiment dans ladite première voie d'écoulement,
**caractérisé en ce que** la soupape comporte un logement (142) de soupape et un organe (160) de soupape mobile dans le logement de soupape, ladite première voie d'écoulement comportant un premier passage d'écoulement dans l'organe de soupape, et ladite deuxième voie d'écoulement comportant un deuxième passage d'écoulement dans l'organe de soupape séparé du premier passage d'écoulement,
ledit logement de soupape comportant en outre un connecteur (210) pour relier ledit conduit de distribution de fluide au logement de soupape,
ledit connecteur possédant une liaison amovible avec le logement de soupape et **en ce que** ledit organe de soupape est un corps monté à coulissement dans une cavité à l'intérieur du logement de soupape, ledit corps présentant une première section (170) d'extrémité, une deuxième section (172) d'extrémité, et une section (174) intermédiaire entre les sections d'extrémité,
ledit premier passage d'écoulement étant situé entre la première section d'extrémité et la section intermédiaire du corps et le deuxième passage d'écoulement étant situé entre la deuxième section d'extrémité et la section intermédiaire du corps.

2. Conteneur de fluide médical selon la revendication 1, dans lequel la soupape est conçue pour pouvoir coulisser sélectivement de manière répétée entre la première position, la deuxième position, et une troisième position qui bloque l'écoulement d'un fluide depuis les compartiments.

3. Conteneur de fluide médical selon la revendication 1 ou 2, dans lequel ladite première voie d'écoulement comporte un premier orifice de sortie dans le réservoir d'un premier côté de la barrière qui correspond au premier compartiment et la deuxième voie d'écoulement comporte un deuxième orifice de sortie dans le réservoir d'un deuxième côté de la barrière qui correspond au deuxième compartiment, dans lequel l'aire d'écoulement du premier orifice de sortie est plus grande que l'aire d'écoulement du deuxième orifice de sortie.

4. Conteneur de fluide médical selon l'une quelconque des revendications 1 à 3, dans lequel le réservoir et la barrière imperméable aux fluides sont sensiblement rigides et autosupportés, la barrière imperméable aux fluides comportant une cloison présentant des joints étanches aux fluides avec les surfaces intérieures du réservoir pour délimiter le premier compartiment d'un côté de la cloison et le deuxième compartiment du côté opposé de la cloison.

5. Conteneur de fluide médical selon l'une quelconque des revendications 1 à 4, comportant en outre un premier orifice (80) d'entrée pour le premier compartiment et un deuxième orifice (82) d'entrée pour le deuxième compartiment de façon à ce qu'un fluide puisse être introduit dans les premier et deuxième compartiments indépendamment.

6. Conteneur de fluide médical selon l'une quelconque des revendications 1 à 5, dans lequel le conteneur est pourvu d'au moins un indicateur (340) indiquant que l'un des compartiments est destiné à contenir un type particulier de fluide médical.

7. Conteneur de fluide médical selon l'une quelconque des revendications 1 à 6, le conteneur étant destiné à une alimentation entérale, le premier compartiment étant conçu pour contenir un aliment liquide, et le deuxième compartiment étant conçu pour contenir du fluide de rinçage, et un premier indicateur (350, 354) indiquant que le premier compartiment est destiné à contenir l'aliment liquide, et un deuxième indicateur (358, 360) indiquant que le deuxième compartiment est destiné à contenir le fluide de rinçage.

8. Conteneur de fluide médical selon l'une quelconque des revendications 1 à 7, comportant en outre des indicateurs (340) de mesure sur au moins un des premier et deuxième compartiments pour indiquer le volume de fluide dans le compartiment.

9. Conteneur de fluide médical selon l'une quelconque des revendications 1 à 8, combiné à un tube (140) de distribution et comportant en outre un support (112) relié au conteneur et conçu pour supporter le conteneur dans une orientation généralement verticale.

10. Conteneur de fluide médical selon l'une quelconque des revendications 1 à 9, dans lequel des composants du conteneur associés au premier et au deuxième compartiments sont codés par couleur selon une couleur attribuée à un fluide médical désigné comme devant être contenu dans les compartiments respectifs.

11. Procédé d'administration de fluides médicaux depuis un conteneur (38) de fluide médical, ledit conteneur comportant un seul réservoir en matériau (40) imperméable aux fluides, le réservoir étant pourvu d'une barrière (60) imperméable aux fluides qui divise le réservoir en au moins un premier et un deuxième compartiment (70, 72) contenant respectivement un premier et un deuxième fluide médical, le procédé consistant à :
pousser une butée (200, 202) pour faire coulisser axialement un corps (160) d'une soupape (134) jusqu'à une première position dans laquelle la soupape permet l'écoulement dudit premier fluide médical depuis le premier compartiment, et dans laquelle la soupape bloque l'écoulement du deuxième fluide médical depuis le deuxième compartiment.

12. Procédé selon la revendication 11, consistant en outre à pousser une butée (200, 202) pour faire coulisser axialement le corps (160) de la soupape (134) jusqu'à une deuxième position dans laquelle la soupape permet l'écoulement dudit deuxième fluide médical depuis le deuxième compartiment, et dans laquelle la soupape bloque l'écoulement dudit premier fluide médical depuis le premier compartiment.

13. Procédé selon la revendication 12, dans lequel ledit premier fluide médical est un aliment liquide et ledit deuxième fluide médical est du fluide de rinçage, ledit procédé consistant en outre à déplacer la soupape jusqu'à ladite première position pour administrer l'aliment liquide à un patient et à déplacer ensuite la soupape jusqu'à ladite deuxième position pour rincer un tube de distribution relié audit orifice de sortie.

14. Procédé selon la revendication 11, consistant en outre à faire coulisser le corps (160) de la soupape (134) dans une cavité (150) à travers une ouverture (164) alignée dans la barrière (60) le long d'un axe formant généralement un angle droit avec un axe central d'un orifice (154) de sortie de la soupape jusqu'à ladite première position.
